Europäisches Patentamt

European Patent Office

Office européen des brevets

(11) Veröffentlichungsnummer: **0 478 929 A1**

## (12) EUROPÄISCHE PATENTANMELDUNG

(21) Anmeldenummer: **91113398.1**

(22) Anmeldetag: **09.08.91**

(51) Int. Cl.⁵: **A61F 2/16**

(30) Priorität: **29.09.90 DE 4030899**

(43) Veröffentlichungstag der Anmeldung:
**08.04.92 Patentblatt 92/15**

(84) Benannte Vertragsstaaten:
**DE ES FR GB IT NL SE**

(71) Anmelder: **IPP INTELLECTUAL PROPERTY PROTECTION AG**
**Bundesstrasse 7**
**CH-6300 Zug(CH)**

(72) Erfinder: **Neuhann, Thomas, Prof. Dr. med.**
**Herzogstrasse 48**
**W-8000 München 40(DE)**

(74) Vertreter: **Liermann, Manfred**
**Schillingsstrasse 335**
**W-5160 Düren(DE)**

(54) **Vorrichtung zur Positionierung einer Intraokularlinse.**

(57) Die Erfindung betrifft eine Vorrichtung zur Positionierung einer Intraokularlinse (14,19) innerhalb des Kapselsackes (25) eines Auges, wobei die Linsenoptik gekrümmte und elastische Stützschlingen (15,20,21) aufweist. Zur Vermeidung einer Überlastung des Kapselsackes (25) wird vorgeschlagen, daß die Stützschlingen (15,20,21) sich in einem für eine Anlage gedachten Bereich zu ihrem Ende hin verjüngen und/oder daß die Stützschlingen (15,20,21) von einem elastischen Kapselsackring (1) umfaßt sind, der in seiner inneren Umfangsfläche eine rillenartige, umlaufende Vertiefung (7) aufweist, in welcher die Stützschlingen (15,20,21) anliegen.

Fig.1

Die Erfindung betrifft eine Vorrichtung zur Positionierung einer Intraokularlinse innerhalb des Kapselsackes eines Auges, wobei die Linsenoptik gekrümmte und elastische Stützschlingen aufweist.

Eine Intraokularlinse ist eine Linse, die üblicherweise nach einer Operation eines grauen Stars als Ersatz der funktionsunfähigen natürlichen Augenlinse in das Auge eingesetzt wird. Hierbei sitzt die Linse in einem Kapselsack, der eine dünne, transparente Membran ist. Der dem vorderen Teil des Auges zugewandte Teil des Kapselsackes wird vordere Kapsel genannt, der hintere Teil wird hintere Kapsel genannt. Hinter der hinteren Kapsel befindet sich die Glaskörper-Grenzmembran. Die Linse fokussiert Lichtstrahlen auf die Retina.

Wenn die physiologische Linse eingetrübt ist, kann sie im Rahmen einer Katarakt-Operation entfernt und durch eine Intraokularlinse ersetzt werden, die die Funktion der physiologischen Linse mindestens teilweise wieder herstellt. Es soll hier ausgegangen werden von einer extrakapsulären Extraktion. Hierzu wird die vordere Linsenkapsel geöffnet und anschließend Linsenkern und Linsenrinde herausgenommen, wobei der Kapselsack im Auge verbleibt. Nach der Entfernung der eingetrübten Linse wird dann die Intraokularlinse in den Kapselsack eingesetzt. Intraokularlinsen sind geometrisch geformte Kunststofflinsen, die aus zwei Teilen bestehen, nämlich der Linsenoptik und der Linsenhaptik. Die Linsenoptik ist eine kleine optische Linse, deren Durchmesser ca. 6 -7 mm betragen kann. Unter Linsenhaptik versteht man die an der Linsenoptik angebrachten Aufhängeteile (Bügel oder Schlingen), die nach der Implantation der Intraokularlinse den optischen Teil in der gewünschten Position halten. Die Haptik dient als Befestigungsvorrichtung im Auge derart, daß sie sich im Gewebe des Auges abstützt. Mit Haptik hat eine Intraokularlinse einen Durchmesser von 10 -14mm, je nach Linsenmodell.

Intraokularlinsen bestehen aus einem durchsichtigen Kunststoff, üblicherweise PMMA (Polymethylmetakrylat). Die Linsenbügel oder Schlingen sind bei heutigen Linsen ebenfalls aus PMMA oder aber aus Polypropylen gefertigt.

Nach extrakapsulärer Extraktion der getrübten Linse, insbesondere wenn das vordere Kapselfenster durch Kapsulorhexis (rundes Aufreißen des vorderen Kapselsackes) erzeugt worden ist, bleibt strukturell ein Kapselsack übrig, in den die Kunstlinse eingpflanzt werden kann.

Postoperativ setzen durch eine Reihe gesetzmäßiger biologischer Vorgänge Schrumpfungsprozesse ein. Bei intaktem zonulärem Aufhängungsapparat der Linse sind diese Schrumpfungsprozesse von klinisch nachrangiger Bedeutung. Ist der Zonular-Apparat jedoch geschwächt (Subluxatio Lentis), wie dies insbesondere bei Pseudoexfoliatio Lentis, aber auch durch Alterungsvorgänge oder etwa bei Retinopathia Pigmentosa, um nur einige Beispiele zu nennen, der Fall ist, so sind diese Schrumpfungsprozesse von klinisch nachteiliger erheblicher Bedeutung. Dabei können Haptiken von Intraokularlinsen, die aus verschiedenen Gründen möglichst flexibel sein sollten, komprimiert werden, so daß die Intraokularlinse disloziert wird.

Mit der WO 89/07426 ist eine Einrichtung der eingangs genannten Art bekannt geworden, bei der in einem künstlichen Kapselsack eine künstliche Linse angeordnet ist. Der künstliche Kapselsack ist hierbei nicht vollständig ausgebildet sondern kann an seiner Vorderseite offen sein. Die künstliche Linse kann mit der Rückseite dieses künstlichen Kapselsackes verbunden oder in diesem mit Hilfe von als Stützschlingen ausgebildeten Haptiken abgestützt sein. Die genauere Form der Stützschlingen ist nicht erkennbar. Aus den Zeichnungen geht hervor, daß die Enden verdickt sind.

Unerwünschterweise muß bei Anwendung dieser Vorrichtung der gesamte natürliche Kapselsack entfernt werden, wodurch zusätzlich die Komplikationsgefahr erhöht wird.

Mit der DE 36 29 193 ist eine künstliche intrakulare Linse bekannt geworden, die gekrümmte und elastische Stützschlingen dort "haptische Schleifen" genannt, aufweist. Diese haptischen Schleifen sollen einen kolbenartigen Endteil (Anspr. 18) und eine veränderliche Querschnittsabmessung aufweisen (Anspr. 19). Diese haptischen Schleifen liegen im Anlagebereich nicht gleichmäßig an und führen daher örtlich zu einer unerwünscht hohen Belastung des Kapselsackes. Faltenbildung am Kapselsack ist unvermeidbar.

Der Erfindung liegt somit die Aufgabe zugrunde, eine Vorrichtung der eingangs beschriebenen Art vorzuschlagen, mit der eine Intraokularlinse sicher und positionsgenau in einem Kapselsack positioniert und gehalten werden kann, ohne daß hierbei der Kapselsack überstrapaziert wird. Gleichzeitig soll die Gefahr der Faltenbildung am Kapselsack vermindert werden.

Diese Aufgabe ist erfindungsgemäß dadurch gelöst, daß die Stützschlingen sich mindestens in einem für eine Anlage gedachten Bereich bis zu ihrem Ende hin verjüngen. Die kolbenartige Ausbildung der Enden der Stützschlingen wird vermieden und es entsteht im Gegensatz zur bisher bekannten punktuellen Anlage eine Anlage über eine weite Strecke am inneren Kapselsackumfang, so daß die Punktbelastung verteilt wird über eine längere Strecke, wodurch die Belastung des Kapselsackes deutlich vermindert wird. Gleichzeitig kann hierdurch die Linsenoptik sicher in Position gebracht und dort gehalten werden. Der sich verjüngende Bereich sorgt dabei gleichzeitig für eine gleichmäßige Anlagekraft entlang der Berührungszone zwischen Haptik und Innenumfang des Kapselsackes. Außerdem kann so weitgehend eine Faltenbildung an der hinteren Kapsel und damit entsprechende Sehstörungen vermieden werden.

Nach einer alternativen Lösung, die ebenfalls von einer Vorrichtung der eingangs beschriebenen Art ausgeht, wird vorgschlagen, daß die Stützschlingen von einem elastischen Kapselsackring aus biokompatiblem Material umfaßt sind, der an seiner inneren Umfangsfläche eine rillenartige, umlaufende Vertiefung aufweist, in welcher die Stützschlingen anliegen. Ein solcher Ring ist in seinem Außendurchmesser auf den Innendurchmesser des Kapselsackes abgestimmt und kann in zusammengelegtem Zustand in den Kapselsack eingebracht werden. Im Kapselsack nimmt er seine kreisförmige Gestalt an und spannt damit den Kapselsack auf, ohne ihn zu belasten. Dies kann insbesondere dann gut erreicht werden, wenn es sich bei den verwendeten Materialien um sogen. Memorymaterialien handelt. Dieser Kapselsackring dient dann als Aufnahmeelement für die Haptik der Intraokularlinse. In der rillenartigen, umlaufenden Vertiefung werden die Stützschlingen eingesetzt. Hierbei ist die gen. Rille in ihren Abmessungen so gehalten, daß die Stützschlinge darin sicher eingesetzt und gehalten werden kann. Hier reicht eine Klemmung der Stützschlingen in der Rille, aber auch die Federkraft der Stützschlingen selbst, die für eine entsprechende Anlagekraft sorgt.
Die hier beschriebene erfindungsgemäße Anordnung hat den weiteren Vorteil, daß die Intraokluarlinse bei Bedarf relativ einfach gegen eine andere Intraokularlinse ausgetauscht werden kann, ohne daß hierfür der Kapselsackring entfernt werden müßte. Außerdem wird auch hier eine Faltenbildung an der hinteren Kapsel sicher vermieden.

Die hier vorgeschlagenen Lösungen haben den weiteren Vorteil, daß der natürliche Kapselsack weitgehend erhalten bleibt, wodurch die Komplikationsgefahr deutlich vermindert wird.

Eine Ausgestaltung der Erfindung sieht vor, daß die die Haptik der Linsenoptik bildenden Stützschlingen einstückig mit der Linsenoptik ausgebildet sind. Dies vereinfacht die Herstellung und die Handhabung der gesamten Intraokularlinse und vermeidet Störungen an der Verbindungsstelle zwischen Haptik und Linsenoptik.

Eine weitere Ausgestaltung der Erfindung sieht vor, daß die Außenseite des Kapselsackringes eine sich mindestens über Teile des Umfanges erstrekkende konkave Ausformung in Form eines äußeren Kanals aufweist. Ein solcher äußerer Kanal kann einen elastischen, ringförmigen, walzenartigen Schwamm aufnehmen, der mit Medikamenten zur protrahierten Abgabe getränkt werden kann. In Frage kommen hierfür z.B. Substanzen, die im Kontakt zytotoxisch wirken und die germinative Zone der Kapselsack-Epithelien, die für den regeneratorischen Nachstar verantwortlich ist, in ihrer Proliferationsfähigkeit hemmen. Auch andere Medikamente, wie antientzündliche Substanzen, können so protrahiert abgegeben werden. Daher sieht auch eine Ausgestaltung der Erfindung vor, daß in diesen äußeren Kanal ein elastischer Schwamm zur Aufnahme von solchen Medikamenten eingesetzt ist.

Eine andere Ausgestaltung der Erfindung wiederum sieht vor, daß der Kapselsackring an mindestens einer Stelle seines Umfanges quer geteilt und mit einem Verschlußmechanismus versehen ist. Hierdurch kann das Einsetzen des Kapselsackringes in den Kapselsack erleichtert werden.

In ergänzender Ausgestaltung der Erfindung ist vorgesehen, daß der Verschlußmechanismus aus zwei Teilen besteht, von denen jeweils eines einstückig an einem durch die Querteilung entstanden Ende des Kapselsackringes ausgebildet ist. In dieser Anordnung kann der Verschlußmechanismus als einfacher Steckverschluß ausgebildet sein, so daß die Handhabung besonders einfach wird.

In erweiternder Ausgestaltung ist noch vorgeschlagen, daß die beiden Teile des Verschlußmechanismus in Längsrichtung verstellbar und in verschiedenen Längsstellungen einrastbar sind. Hierdurch wird eine Durchmesserverstellung des Kapelsackringes möglich, so daß der Kapselsackring in seinem Durchmesser an unterschiedliche Durchmesser des Kapselsackes angepaßt werden kann.

Ergänzend ist dann weiter nach der Erfindung vorgesehen, daß ein Teil des Verschlußmechanismus mindestens einseitig eine radial nach außen oder innen gerichtete sägezahnartige Kontur aufweist, die sich mindestens über einen Teil der Dicke des Ringes erstreckt, während der andere Teil in entsprechender Dicke und Länge in Längsrichtung eine Ausnehmung aufweist, die mindestens einseitig eine zugeordnete sägezahnartige Innenkontur als Gegenkontur aufweist, wobei Innenkontur und Außenkontur einander zugeordnete Verschlußzähne bilden. Ein solcher Verschlußaufbau ist einfach herstellbar und zuverlässig in der Funktion. Er kann z.B. vor oder nach dem Einsetzen auf kleinsten Durchmesser zusammengeschoben und erst im Kapselsack auf den richtigen Durchmesser auseinandergezogen werden.

Schließlich ist nach einer anderen Ausgestaltung der Erfindung noch vorgeschlagen, daß die Schlingen einen rechtwinkligen Querschnitt aufweisen. Ein solcher Querschnitt ist einfach herstellbar und es kann durch kontinuierliche Querschnittsveränderung leicht ein bestimmtes Federverhalten der Schlinge erzeugt werden.

Die Vorrichtung soll nun anhand der beigefügten Zeichnungen näher erläutert werden.

Es zeigen:

Figur 1    Kapselsackring von oben gesehen

Figur 2    Verschlußmechanismus des Kapselsackrings von oben gesehen

Figur 3    Detail-Darstellung des Verschlußteils des Kapselsackringes

Figur 4    Querschnitt des Kapselsackringes entlang der Linie I-I nach Fig. 1.

Figur 5    Segment des Kapselsackringes in perspektivischer Darstellung mit eingelegtem Medikamentenschwämmchen.

Figur 6    Schnitt entlang der Linie II-II nach Fig. 3

Figur 7    Schnitt entlang der Linie III-III nach Fig. 3

Figur 8    Perspektivische Darstellung des unteren Verschlußteils des Kapselsackringes

Figur 9    Segment des Kapselsackringes in perspektivischer Darstellung mit eingesetzter Haptik einer Intraokularlinse

Figur 10    Intraokularlinse mit konisch auslaufenden Schlingen in Draufsicht

Figur 11    Endstück einer konisch auslaufenden Schlinge

Figur 12    Endstück einer konisch auslaufenden Schlinge mit rechtwinkligem Querschnitt.

Figur 14    Pinzette mit einem zur Implantation zusammengedrückten Kapselsackring

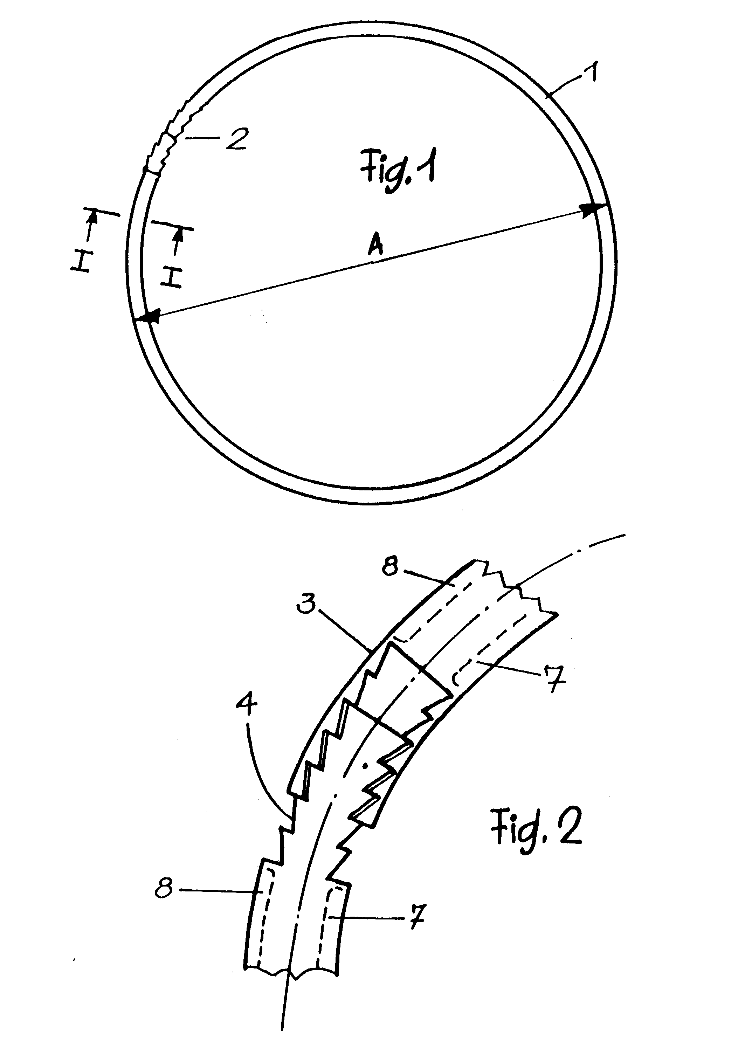

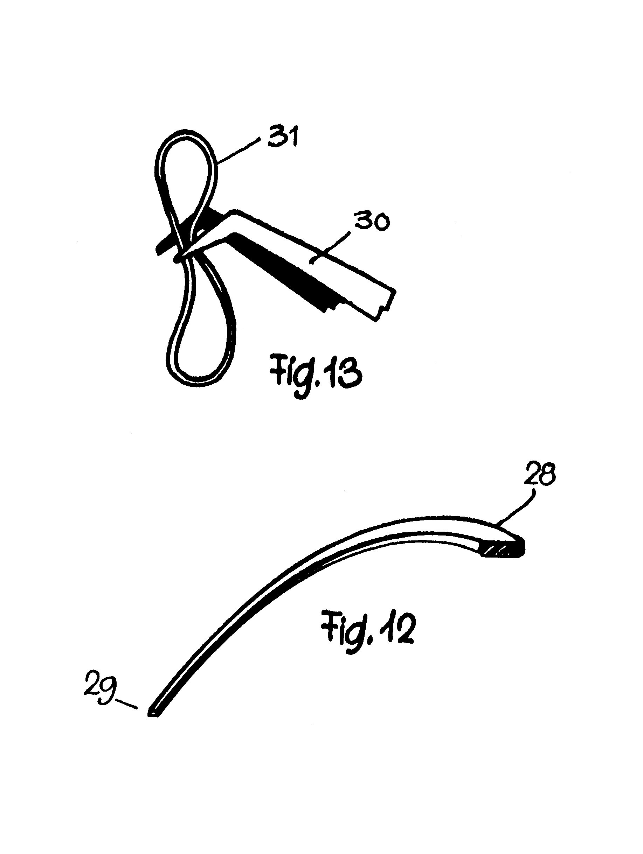

Figur 1 zeigt den Kapselsackring 1 in Draufsicht mit Verschlußmechanismus 2. Über den Verschlußmechanismus läßt sich die Größe des Kapselsackringes 1 variieren. Hierbei kann der Durchmesser A des Ringes um ca. 3 mm verändert werden. Der Einstellbereich reicht von 9 mm bis 12 mm. Die Mitteneinstellung von 10,5 mm entspricht dem Normaldurchmesser. Für besonders große oder besonders kleine Augen werden Ringe mit anderen Durchmessern verwendet. Der Verschlußmechanismus 2 ist ratschenförmig aufgebaut. Figur 2 zeigt eine Draufsicht des Verschlußmechanismus. In das Unterteil 3 des Verschlußmechanismus sind christbaumartig angeordnete oder sägezahnartig angeordnete Kerben eingebracht, in denen das Gegenstück des Oberteils 4 des Verschlußmechanismus 2 eingesetzt ist. Der Ring wird z.B. in völlig geschlossenem Zustand in den Kapselsack 25 implantiert. Dazu wird er mit einer Pinzette 30 oder einem anderen geeigneten Instrument zusammengedrückt, wie dies in Fig. 13 dargestellt ist. Der Kapselsackring 1 verformt sich dabei zu einem schmalen, länglichen Gebilde 31. Nach Einsetzen in den Kapselsack 25 wird der Durchmesser A des Kapselsackringes 1 mit Hilfe eines geeigneten Instrumentes auf die gewünschte Größe auseinandergezogen. Dabei bewegt sich das Oberteil 4 des Verschlusses aus dem Unterteil 3 in Richtung des Pfeils B heraus (Fig.2).

Nach einigen Zehntel Millimetern rastet das Oberteil 4 in einer neuen Stellung im Unterteil 3 ein. Fig. 2 zeigt als Beispiel eine Rasterstellung, nach dem der Verschluß um zwei Zähne und damit zwei Rasterstellungen auseinandergezogen wurde. Fig. 3 zeigt die gleiche Situation in Seitenansicht vom Zentrum des Ringes aus radial nach außen gesehen. Zwei Verschlußzähne 5 sind hierbei aus dem Unterteil 3 herausgetreten. Der Kapselsackring 1 kann über den Verschlußmechanismus 2 dabei max. so weit geöffnet werden, daß noch ein Einrastglied oder Verschlußzahn 5 des Oberteils 4 im Unterteil 3 verbleibt, um einen sicheren Halt zu gewährleisten. Den Verschlußzähnen 5 entsprechen dabei die Verschlußzähne 6 im Unterteil 3.

Der Kapselsackring 1 weist auf seiner radialen nach innen gerichteten Seite eine als Rille ausgebildete umlaufende Vertiefung 7 auf, die jeweils erst am Unterteil 3 bzw. am Oberteil 4 in Umfangsrichtung endet. Diese umlaufende Vertiefung 7 dient der Aufnahme der Linsenschlingen 15 bzw. 20 und 21, dient also der Aufnahme der Haptik der Linsenoptik. Die umlaufende Vertiefung 7 ist nur geringfügig weiter als eine Linsenschlinge und tief genug, um einen sicheren Sitz nach einem Einspringen der Schlinge zu garantieren. Aufgrund der Elastizität der Schlingen sind diese immer bestrebt, sich radial nach außen aufzuweiten, so daß sie aufgrund dieser Elastizität immer sicher in der umlaufenden Vertiefung 7 halten. Die umlaufende Vertiefung 7 kann jedoch in ihren Querschnittabmessungen durchaus so bemessen sein, daß die Schlingen nach einem Einspringen in die umlaufende Vertiefung 7 dort leicht geklemmt werden.

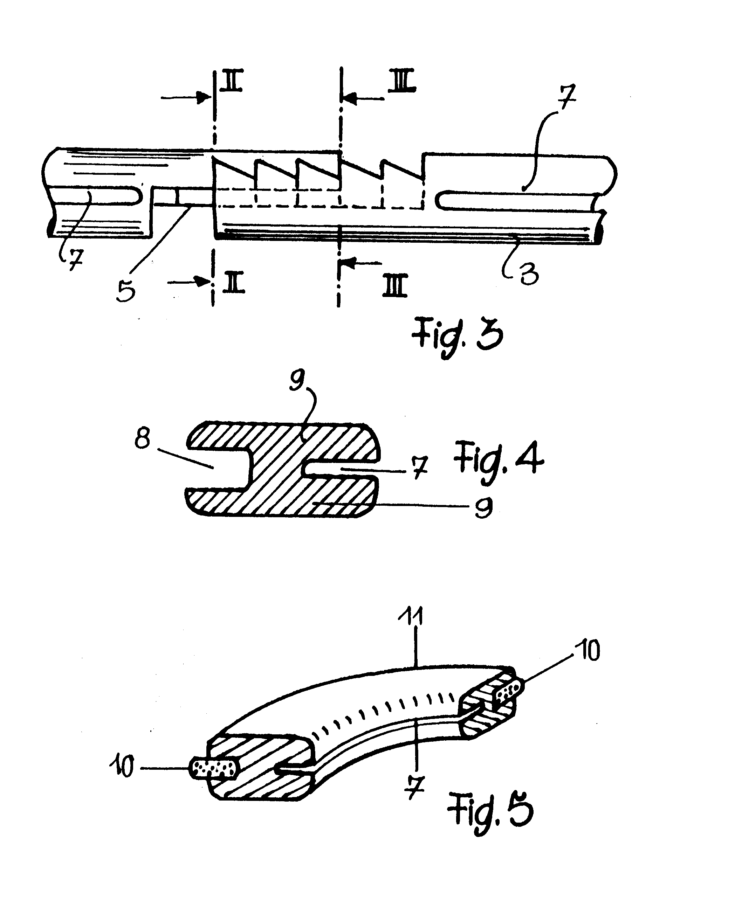

Figur 4 zeigt einen Querschnitt durch den Kapselsackring 1, nach der Schnittlinie I-I in Fig. 1. Der Querschnitt 9 ist nahezu rechteckig mit abgerundeten Querschnittsecken. Im Querschnitt ist einerseits die umlaufende Vertiefung 7 und andererseits der umlaufende äußere Kanal 8 erkennbar. Die Dicke des Querschnittes kann hierbei z.B. 0,5 mm und die Breite des Querschnittes z.B. 2,0 mm betragen.

In Figur 5 ist perspektivisch ein Ringsegment 11 dargestellt, in welchem der im äußeren Kanal 8 eingesetzte Aufnahmekörper 10 als Schwamm ausgebildet ist zur Medikamentenaufnahme erkennbar ist. Auf der Innenseite ist wieder die umlaufende Vertiefung 7 zur Aufnahme von Linsenschlingen 15 bzw. 20 und 21 erkennbar.

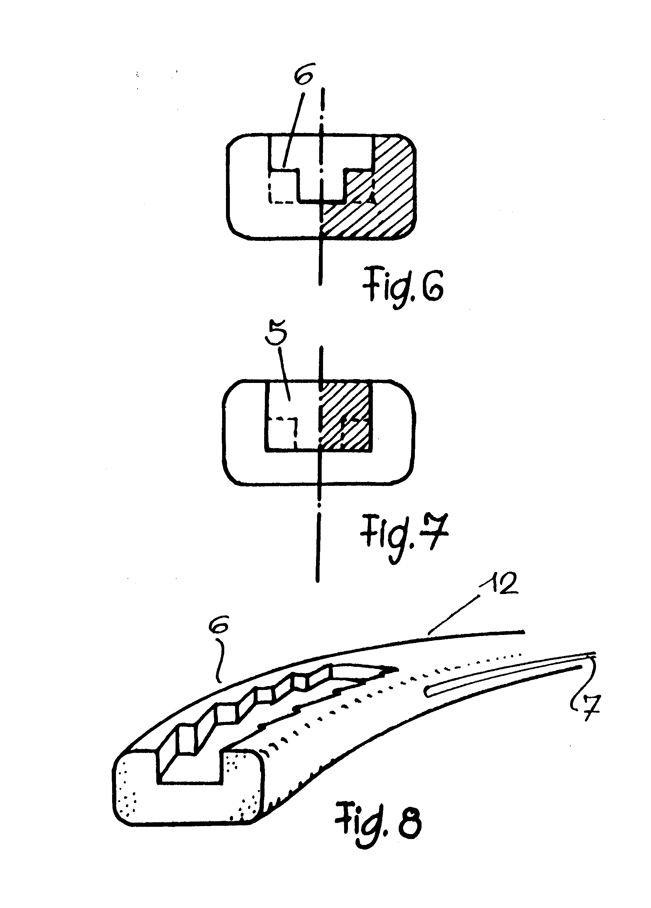

Figur 6 zeigt einen Schnitt entlang der Linie II-II nach Bild 3. In dieser Darstellung wird deutlich, daß die unteren Verschlußzähne 6 etwa ein Drittel der Gesamthöhe des Kapselsackringes 1 ausmachen.

Figur 7 zeigt einen Schnitt entlang der Linie III-III nach Figur 3. Auch hier nehmen die Verschlußzähne 5 etwa ein Drittel der Gesamthöhe des Kapselsackringes 1 ein.

Figur 8 zeigt den unteren Teil des Verschlußmechanismus 2 in perspektivischer Ansicht. Gut erkennbar sind hierbei die am Verschlußende 12 vorgesehenen und nach außen offenen Verschlußzähne 6. Ebenfalls gut erkennbar ist der Beginn der umlaufenden Vertiefung 7 zur Aufnahme der Linsenschlingen.

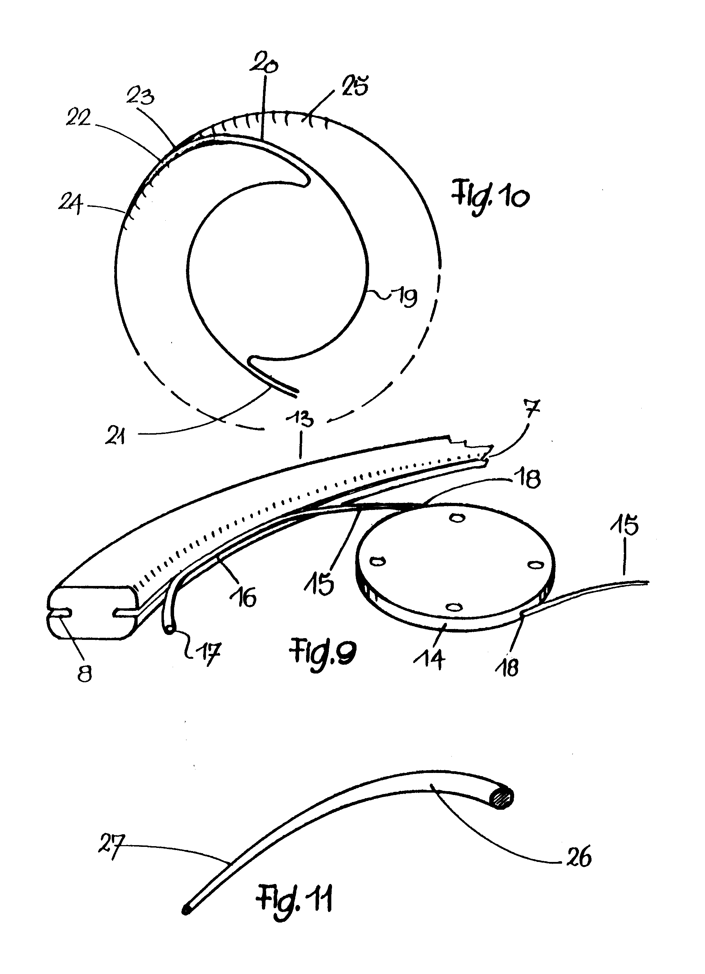

Figur 9 zeigt ebenfalls ein Segment 13 des Kapselsackringes 1 in perspektivischer Darstellung. Auch hier ist wieder der Verlauf der umlaufenden Vertiefung 7 sowie die Lage des äußeren Kanals 8 gut erkennbar. In Figur 9 ist weiter eine Intraokularlinse 14 dargestellt, deren eine Schlinge 15 an der Stelle 16 in die umlaufende Vertiefung 7 des Kapselsackringes 1 eingelegt ist. Die Schlinge 15 paßt dabei gut und tief genug in die umlaufende Vertiefung 7, so daß ein sicherer Halt gewährleistet wird und damit die Linse 14 gut abgestützt wird. Das Schlingenende 17 reicht aus dem Kapselsackring wieder etwas heraus. Zur Entnahme der Schlinge 15 aus der umlaufenden Vertiefung 7 kann mit einem geeigneten Instrument, bspw. einer Pinzette, das Schlingenende 17 gefaßt werden und in Richtung der Intraokularlinse 14 gezogen werden. Dabei tritt an der Stelle 16 die Schlinge wieder aus der umlaufenden Vertiefung 7 aus und ist damit vom Kapselsackring befreit.

In der Darstellung nach Figur 9 ist eine Intraokularlinse 14 dargestellt mit eingesetzten Prolene-Schlingen, die aus dem optischen Körper an der Stelle 18 austreten. In der Darstellung nach Figur 9 weist die Linsenoptik darüberhinaus hier nicht näher bezeichnete Bohrungen auf, die der Manipulation der Linsenoptik dienen, im Rahmen der Erfinnung jedoch keine besondere Bedeutung haben.

Die Schlingen 15 der Intraokularlinse 14 sind bspw. aus Prolene oder aus extrudiertem PMMA gefertigt. Bei Figur 9 liegt eine J-förmige Schlinge vor, die in ihrem Durchmesser vom Ansatzpunkt 18 bis zu ihrem Endpunkt 17 konstant ist. Eine Modifikation dieser Schlingenform zeigt Figur 10. Die dort vorgesehene Intraokularlinse 19 hat ebenfalls zwei Schlingen 20 und 21. Die Schlinge 21 ist hier nur angedeutet aber nicht weiter dargestellt. Der Durchmesser der Schlinge 20 ändert sich in Höhe der Stelle 22 dahin, daß er zum Ende der Schlinge 20 hin immer geringer wird. Da das Schlingenmaterial elastisch ist, hat dies zur Folge, daß sich die Schlinge 20 im Normalfall nicht nur an einer Stelle 22 bei der Implantaion in einen Kapselsack 25 anlegt, sondern daß durch die Verjüngung der Schlinge und deren Tendenz nach außen aufspringen zu wollen, eine Anlagefläche von der Stelle 23 bis zur Stelle 24 existiert. Hierdurch wird eine punktförmige Belastung und damit eine Überlastung des Kapselsackes vermieden und der Linsenoptik ein sicherer Halt verliehen. Gleichzeitig wird ein Verspannen des Kapselsackes verhindert.

Herkömmliche Schlingen verhalten sich wie in Figur 9 mit der Schlinge 15 der Intraokularlinse 14 gezeigt. Sie stützen sich an einer Stelle 16 ab und laufen dann von dieser Stützstelle wieder weg, um bei 17 zu enden. Im Ausführungsbeispiel der Figur 9 kann dies geduldet werden, da die entsprechende punktförmige Belastung von dem erfindungsgemäßen Kapselsackring aufgenommen wird. Durch die Verwendung des Kapselsackringes wird es also möglich, auch die herkömmlichen Bauarten der Intraokularlinsen weiter zu benutzen, die bei einem Direkteinsatz in den Kapselsack diesen auf Dauer überlasten und verspannen würden, was mit der erfindungsgemäßen Vorrichtung vermieden werden kann.

Der Kapselsackring 1 kann natürlich auch dann verwendet werden, wenn eine Intraokularlinse 19 mit den Schlingen 20 und 21 verwendet wird. Die Schlingen 20 und 21 liegen dann einfach in der umlaufenden Vertiefung 7 an und werden dort sicher gehalten. Figur 11 zeigt hierzu nochmals einen Abschnitt einer Prolene-Schlinge, die nach dem Austritt aus dem Linsenkörper für eine gewisse Strecke einen konstanten Durchmesser 26 aufweist, um sich dann im weiteren Verlauf zu ihrem Ende hin zu verjüngen bis zum verjüngten Ende 27. Prolene-Schlingen oder auch Schlingen aus extrudiertem PMMA werden separat in den optischen Teil einer Intraokularlinse eingesetzt. Es ist aber auch möglich, Intraokularlinsen aus PMMA zu fertigen, deren Haptiken bei der Fertigung der Linse sofort am Linsenkörper einstückig mit diesem ausgebildet werden. Die Haptiken solcher Linsen haben meist einen rechteckigen Querschnitt 28, wie dies in Figur 12 dargestellt ist. Die Schlinge nach Figur 12 ist ebenfalls in Richtung ihres Endes 29 hin verjüngt, wobei die Verjüngung an beiden Achsen des rechtwinkligen Querschnitts erfolgen kann. Der Kapselsackring kann natürlich auch in geöffnetem Zustand in den Kapselsack eingesetzt und dann erst geschlossen werden. Dies kann in bestimmten Fällen das Einsetzen erleichtern.

Liste der verwendeten Bezugszeichen

| | |
|---|---|
| 1. | Kapselsackring |
| 2 | Verschlußmechanismus |
| 3 | Unterteil |
| 4 | Oberteil |
| 5 | Verschlußzähne |
| 6 | Verschlußzähne |
| 7 | umlaufende Vertiefung |
| 8 | äußerer Kanal |
| 9 | Querschnitt |
| 10 | Aufnahmekörper |
| 11 | Ringsegment |
| 12 | Verschlußende |

13    Segment
14    Intraokularlinse
15    Schlinge
16    Stelle
17    Schlingenende
18    Stelle
19    Intraokularlinse
20    Schlinge
21    Schlinge
22    Stelle
23    Stelle
24    Stelle
25    Kapselsack
26    konstanter Durchmesser
27    verjüngtes Ende
28    rechtwinkliger Querschnitt
29    Ende
30    Pinzette
31    längliches Gebilde
A     Durchmesser
B     Pfeil

## Patentansprüche

1. Vorrichtung zur Positionierung einer Intraokularlinse innerhalb des Kapselsackes eines Auges, wobei die Linsenoptik gekrümmte und elastische Stützschlingen aufweist, dadurch gekennzeichnet, daß die Stützschlingen (20,21) sich mindestens in einem für eine Anlage gedachten Bereich (23-24) bis zu ihrem Ende (24) hin verjüngen.

2. Vorrichtung mindestens nach dem Gattungsbegriff des Anspruchs 1, dadurch gekennzeichnet, daß die Stützschlingen (20,21) von einem elastischen Kapselsackring (19) aus biokompatiblem Material umfaßt sind, der an seiner inneren Umfangsfläche eine rillenartige, umlaufende Vertiefung (7) aufweist, in welcher die Stützschlingen (20,21,15) anliegen.

3. Vorrichtung nach einem der Ansprüche 1 und 2, dadurch gekennzeichnet, daß die die Haptik der Linsenoptik bildenden Stützschlingen (20,21) einstückig mit der Linsenoptik ausgebildet sind.

4. Vorrichtung nach einem der Ansprüche 1 bis 3, dadurch gekennzeichnet, daß die Außenseite (Fornix-Seite) des Kapselsackringes (1) eine sich mindestens über Teile des Umfanges erstreckende konkave Ausformung in Form eines äußeren Kanals (8) aufweist.

5. Vorrichtung nach Anspruch 4, dadurch gekennzeichnet, daß in dem äußeren Kanal (8) ein Aufnahmekörper (10) zur Aufnahme von Medikamenten eingesetzt ist.

6. Vorrichtung nach einem der Ansprüche 1 bis 5, dadurch gekennzeichnet, daß der Kapselsackring (1) an mindesten einer Stelle seines Umfanges quer geteilt und mit einem Verschlußmechanismus (2) versehen ist.

7. Vorrichtung nach einem der Ansprüche 1 bis 6, dadurch gekennzeichnet, daß der Verschlußmechanismus (2) aus zwei Teilen (3,4) besteht, von denen jeweils eines einstückig an einem durch die Querteilung entstandenen Ende des Kapselsackringes (1) ausgebildet ist.

8. Vorrichtung nach Anspruch 7, dadurch gekennzeichnet, daß die beiden Teile (3,4) des Verschlußmechanismus (2) in Längsrichtung verstellbar und in verschiedenen Längsstellungen einrastbar sind.

9. Vorrichtung nach Anspruch 8, dadurch gekennzeichnet, daß ein Teil (3,4) des Verschlußmechanismus (2) mindestens einseitig eine radial nach außen oder innen gerichtete sägezahnartige Kontur aufweist, die sich mindestens über einen Teil der Dicke des Ringes erstreckt, während der andere Teil in entsprechender Dicke und Länge in Längsrichtung eine Ausnehmung aufweist, die mindestens einseitig eine zugeordnete sägezahnartige Innenkontur als Gegenkontur aufweist, wobei Innenkontur und Außenkontur einander zugeordnete Verschlußzähne (5,6) bilden.

10. Vorrichtung nach einem der Ansprüche 1 bis 9, dadurch gekennzeichnet, daß die Schlingen (20,21) einen rechtwinkligen Querschnitt (28) aufweisen.

11. Vorrichtung nach Anspruch 5, dadurch gekennzeichnet, daß der Aufnahmekörper (10) ein elastischer Schwamm ist.

Fig.1

Fig.2

Fig. 3

Fig. 4

Fig. 5

Fig. 6

Fig. 7

Fig. 8

Fig. 10

Fig. 9

Fig. 11

Fig.13

Fig. 12

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y<br>A | US-A-4 638 056 (CALLAHAN ET AL.)<br> (* zusammenfassung * )<br>* Abbildungen * *<br>– – – | 1,3<br>2 | A 61 F 2/16 |
| Y | EP-A-0 246 216 (PRECISION-COSMET CO.)<br>* zusammenfassung *<br>* Abbildung 1 * *<br>– – – | 1,3 | |
| A,D | WO-A-8 907 426 (LANGERMAN)<br>* Seite 21, Zeile 18 - Zeile 30 *<br>* Abbildungen 7-10 * *<br>– – – | 2 | |
| A | WO-A-8 701 931 (TILLAY)<br>* zusammenfassung *<br>* Abbildungen 14,16 * *<br>– – – | 1 | |
| A | US-A-4 556 998 (SIEPSER)<br>* Abbildung 2A * *<br>– – – – – | 1 | |

RECHERCHIERTE SACHGEBIETE (Int. Cl.5)

A 61 F

**Der vorliegende Recherchenbericht wurde für alle Patentansprüche erstellt**

| Recherchenort | Abschlußdatum der Recherche | Prüfer |
|---|---|---|
| Den Haag | 07 Januar 92 | GODOT T.G.L. |